# EUROPEAN PATENT SPECIFICATION

(11) **EP 1 708 725 B1**
(45) Date of publication and mention of the grant of the patent: **04.05.2016**
(21) Application number: 04802161.2
(22) Date of filing: 07.12.2004
(51) Int. Cl.: A61K 36/28, A61K 36/185, A61K 31/00, A61K 35/644, A61K 45/06

(54) **THERAPEUTICAL COMPOSITION FOR THE TREATMENT OF DERMATOSIS COMPRISING AN EXTRACT OF CALENDULA OFFICINALIS AND HYPERICUM PERFORATUM**
THERAPEUTISCHE ZUSAMMENSETZUNG ZUR BEHANDLUNG VON DERMATOSEN MIT EINEM EXTRAKT AUS CALENDULA OFFICINALIS UND HYPERICUM PERFORATUM
COMPOSITION THERAPEUTIQUE POUR LE TRAITEMENT DE LA DERMATOSE RENFERMANT UN EXTRAIT DE CALENDULA OFFICINALIS ET D'HYPERICUM PERFORATUM

(30) Priority: 31.12.2003 BG 10851103
(43) Date of publication of application: 11.10.2006
(73) Proprietor: Naydenov, Vladimir Yosifov, 1303 Sofia (BG); Kurdov, Kiril Dimitrov, 1379 Sofia (BG)
(72) Inventor: Naydenov, Vladimir Yosifov, 1303 Sofia (BG); Kurdov, Kiril Dimitrov, 1379 Sofia (BG)
(74) Representative: Georgieva, Lilia Tzvetkova
(86) International application number: PCT/BG2004/000024
(87) International publication number: WO 2005/063266

(56) References cited:
- WO-A-91/15218
- WO-A-97/42963
- GB-A- 2 164 253
- RU-C1- 2 099 410

## Description

The present invention relates to a therapeutical composition for use in the treatment of dermatosis such as psoriasis, neurodermatitis, trophic sores, eczemas, acne and other disorders of the skin, which has anti-inflammatory, regenerative and trophic effect and normalizes the metabolism in the dermal and epithelial tissues.

### BACKGROUND OF THE INVENTION

It is known that in the treatment of dermatitis, in particular psoriasis, extracts from different combinations of medicinal herbs are used, which have anti-inflammatory, regenerative and bactericidal action.

WO91/15218 describes a therapeutical composition against psoriasis based on an extract of a combination of the herbs Chelidonium L. and Hypericum I., and optionally Bardanum L., Calendula L., Achilea L. and Matricaria Chamomilla L. More specifically the composition contains acetone extract of the herbs, included in different carriers for external application.

US 6225342 and US 6313099 describe the use of sesquiterpene glucosides, isomers and other derivatives thereof from Calendula L. for the treatment of disease involving cell hyperproliferation, psoriasis in particular, obtained by multiphase extraction of Calendula species.

WO 02/051427 describes a stabilized extract of Hypericum perforatum L. and its use in the treatment of disease involving cell proliferation and inflammation, psoriasis in particular. The extract has a lower chlorophyll and pro-anthocyanidinen content and contains hyperforin, hypericin and flavon, as well as a stabilizer selected from ester of ascorbinic acid, thiolic compounds, cysteine, glutathione, etc. The extract contains essential oils, carotenoids, anthraquinone derivatives, flavonoides, catechins and epicatechins, tan compounds, phenolic acids, sterols, wax, paraffin, in specific quantities.

Despite the multitude of known therapeutical compositions, the problems in treating dermatosis, in particular psoriasis, still exist because these known drugs solve separate aspects of the disease. There is a need for improvement of the known drugs, effective in individual cases, by taking into account the basic factors related to the disease, the prime causes and the intimate mechanism of the anomalies in the diseased cells.

### DISCLOSURE OF THE INVENTION

An object of the present invention is to provide a therapeutical composition for use in the treatment of dermatosis which ensures complex intervention and acts on the anomalies to regulate the metabolism, in order to restore the structure and the function of the dermis tissues.

The present invention provides a therapeutical composition for use in the treatment of skin diseases, in particular psoriasis, which contains an extract of the medicinal herbs Calendula officinalis L. and Hypericum perforatum L. in ratio 1:1 to 3:1 parts by weight. The extract contains: low-molecular peptides and free amino acids to a total amount of 0.5-6.0 % by weight of the extract dry mass, more specifically histidine, cystine, serine, alanine, valine, leucine, isoleucine, phenylalanine, glycine, lysine, proline, tyrosine, glutamine, asparagine, methionine; unsaturated fatty acids 1.0-10.0 % by weight of the extract dry mass, more specifically linoleic acid, linolenic acid and oleic acid; vitamins to a total amount of 1.0-6.0 % by weight of the extract dry mass, including retinoides 0.6-3.0 %; and minerals and microelements 1.0-6.0 % by weight of the extract dry mass.

Besides retinoides, other vitamins contained in the extract according to the present invention are the wide range of vitamins of group B, vitamins C, D, E, P, H.

The group of minerals and microelements contained in the extract includes: Fe³⁺, Mg²⁺, Mn²⁺, K⁺ Na⁺, Zn²⁺, sulphur, phosphor, calcium.

Several enzymes activities such as kinases, oxidases, catalases, phosphatases, proteolytic enzymes are detected in the extract.

Products of nucleoproteins decomposition were found in the amount of 0.1-0.8 % by weight of the extract dry mass, which include: amino acids formed by hydrolysis of histones such as cysteine; phosphoric acid; nucleosides; ribose; purines; and pyrimidines, which assist in cell metabolism.

The extract also contains other active components known from the prior art. For example, an extract of Calendula officinalis L. contains: triterpene saponins, calendulosides, oleanolic acid derivatives, lupeol derivatives, sesquiterpenes - calenduline and derivatives thereof, essential oil, carotenoids, flavonoides, tan compounds and mucous compounds, phytosterols, phytohormones, organic acids, alkaloids, phitoncides. An extract of Hypericum perforatum L. contains: condensed anthraquinone derivatives (hypericin), essential oils, carotenoids, sesquiterpenes, flavonoides, biflavons, monomer catehins and epicatehins, tan compounds - tannins, hyperforins, phenolic acids (chlorogenic acid), sterols, waxes, paraffins.

Proceeding from the biochemistry and the pathology of dermatosis, in particular psoriasis, and the need for complex intervention, a composition of an extract of medicinal herbs was created which acts on the anomalies to regulate the metabolism, in order to restore the structure and function of the dermis tissues. To this end, in the process of directed extraction the macromolecular compounds are reduced to low-molecular fragments and constituent elements, where partial physicochemical transformations occur among them in a natural ionobalanced medium.

In the extract according to the present invention are found products of the chlorophyll decomposition in a quantity 0.3-1.5% of the extract dry mass, which include phytol, magnesium, chlorophyll porphyrins. Practically no chlorophyll in the extract is detected (HPLC analysis).

The extract contains also compounds such as cycloheptanon and troponon with cytostatic action regulating hyperproliferation, as well as thiolic compounds.

The high degree of hydrolysis of the polypeptides contributes for their nearly complete assimilation by the dermis cells. They were found in a step-by-step synthesis of scleroproteinic structures in the corneous layer of the epidermis, composed mainly of keratinic proteins, with 14-19% content of the amino acid cystine, which is synthesized in the presence of methionine, serine and retinoides as regulator. The disulphidic bonds between the opposite cystinic molecules ensure the stability of the keratins. Another determining factor for the formation of the scleroproteinic structures is the essential amino acid histidine, which is dominant in the formation of the granular layer of the epidermis. The essential amino acids, the unsaturated fatty acids, the vitamins, including retinoides, the microelements and other biologically active substances mentioned above have a specific effect on the trophics and metabolism regulation. They take part in the biosynthesis of high energy compounds - nucleosidephosphates; phospholipids; proteoglycans, and in the metabosilm of arachidonic acid and prostaglandins.

The presence of the free amino acids cystine, histidine, serine, methionine, glycine, proline, lysine, as well as retinoides, vitamins C, B and D and iron (Fe³⁺) in the extract according to the present invention provides the basic constructive factors for the keratins, collagen and elastin. On the other hand, the cystine, serine, tyrosine and histidine content in combination with the electrophilic groups of Mg²⁺, Mn²⁺, Fe³⁺ , K⁺, Na⁺, Zn²⁺ are the basic constructive factors for the enzymes active center. The functional groups of the serine, tyrosine, cysteine sulphhydryl group and particularly of the histidine imidazole group contained in the extract, which have a nucleophilic activity, in combination with the electrophilic groups of Mg²⁺, Man²⁺, Fe³⁺ and the NH₃⁺ groups of the proteids are the basic constructive factors for the enzymes catalytic centers. Vitamins B1, B2, B3, B5, and the metal ions Zn²⁺, K⁺, Na⁺ participate as co-factors for many enzymes.

The complex extraction of Calendula officinalis L. and Hypericum perforatum L. in the above mentioned ratio produces an extract with multilateral modifications and biotransformations in a natural biological medium, which determines a different composition of the extract compared with the prior art and the obtaining of a pharmacological and pharmaco-kinetic effect significantly higher than in the separate application of the two extracts.

In one embodiment of the invention, in order to enhance the anti-inflammatory, painkilling, anti-itching, hyperproliferation-normalizing and/or sore-healing effect, the extract may contain additionally extractions of at least one of the medicinal herbs selected from the group: Lycopodium Clavatum L., Matricaria chamomilla L., Ruta glaveolens L., Urtica dioica L., Uncaria tomentosa, Achillea millefolium L., Melissa officinalis L., Chelidonium majus L., Salvia officinalis L., Thymus sp. diversa, Taraxacum officinale Web., Genista tinctoria L., Alloe arborescens Mill, Uncaria tomentosa, Cotinus coggyrgia, Juglans regia L., Allium sativum L., Equisetum arvense L., Crataegus monogyna Jacq., Plantago lanceolata L., Symphytum officinale L., Juniperus communis L., Pithecolobium unguiscati, Hedera helix L. The ratio of these medicinal herbs to the total mass content of Calendula officinalis L. and Hypericum perforatum L. is determined so as the quantities of low-molecular peptides and free amino acids, fatty acids, vitamins, minerals and microelements in the end extract would be higher than the lower limit of the above indicated quantities of these substances.

For example, an extract of Licopodium Clavatum L. in a quantity 15-35% of the total mass of Calendula officinalis L. and Hypericum perforatum L. added to the basic extract strengthens the curative effect of the main extract in cases of hyperproliferation, slowing down and regulating cell hyperproliferation, and also has an anti-itching effect.

In another embodiment of the invention, to the basic extract of Calendula officinalis L. and Hypericum perforatum L. is added an extract of at least one apian product: honey, royal jelly, beeswax, bees' pollen, propolis, in a quantity 10-25% by weight of the basic extract of Calendula officinalis L. and Hypericum perforatum L. The apian products content in the extract widens the range of amino acids, enzymes, microelements and vitamins. They have a trophic, anti-inflammatory and metabolism-regulatory action.

The method for preparing the extract according to the invention is the following:
30 to 70% fresh plant material from Calendula officinalis L. and Hypericum perforatum L. is used and dried plant material for the remaining percentage up to 100%. The dried plant material is of particle size from 1 to 10 mm, and the fresh one - from 10 to 20 mm. The extraction is carried out with water, organic solvent and organic food oils, e.g. Olium olivarum (olive oil), in a herb:solvent ratio from 1:5 to 1:25. During the extraction constant contact between the components is ensured by optimum stirring.

The extraction is carried out at a temperature between 10°C and 78°C with duration as follows:
- Water extraction: 1/2 of the plant material intended for extraction is extracted with water in a ratio 1:6 for a period of 20 days at a temperature of 18°C. The extract is decanted and 5 parts by weight of water are added to the residue. The extraction is carried out in 2 to 4 phases of total duration about 50 days, and at the phase borders the extract is stabilized with known preservatives and stabilizers such as esters of parahydrobenzoic acid, nipagin, nipazol. The extracts are mixed, filtered, stabilized and concentrated.
- Extraction with organic solvent (chloroform, hexane, ether, ethanol, acetone): 1/4 of the plant material intended for extraction is extracted with 30 % ethanol in a ratio 1:6 for 24 to 48 hours at a temperature of 25°C. The extract is decanted and the residue is further extracted with 4-6 parts by weight of 45 % ethanol by means of a reflux condenser for 6 to 12 hours at a temperature of 78°C. The two alcoholic extracts are filtered, mixed and concentrated under vacuum and stabilized.
- Oil extraction: 1/4 of the plant material intended for extraction is extracted with vegetable oils such as olive oil in a ratio 1:5 for 5 days at a temperature of 30°C, 5 days at a temperature of 25°C, and 5 days at a temperature of 20°C. The oil extract is stabilized with known antioxidants.

The obtained water extract, oil extract and organic solvent extract are mixed.

The extract according to the invention may be obtained by other known methods, which ensure a content of low-molecular peptides, free amino acids, unsaturated fatty acids, vitamins, minerals and microelements in the quantities specified above.

The composition for use in the treatment of skin diseases according to the invention can be used in the following pharmaceutical formulations: unguent, cream, emulsion, gel, shampoo. When it is for internal application in the form of emulsion, sterile filtration of the extract is done.

As ointment base in the unguent is used liquid paraffin, glycerin stearate, vaseline, lanolin, white wax, cetaceum in a ratio from 9:1 to 15:1 to the extract quantity. The ointment base provides maximum percutaneous resorption of the biologically active substances contained in the medicinal herb extract. The unguent may also contain surface- active agent, as well as preservatives and stabilizers, for example sodium benzoate, nipagin, potassium sorbate.

Emulsion of the extract according to the invention is obtained by subjecting the extract to sterile filtration and then corrigence and stabilizer are added.

To obtain gel, the extract is filtered and diluted, then known preservatives and stabilizers are added in a ratio 1.5-4.0 parts by weight to 100 parts by weight of the extract, and also antioxidants, polymers and activators from 0.6 to 1.6 parts by weight.

The composition according to the invention is used for the treatment of dermatosis such as psoriasis, neurodermatitis, trophic sores, eczemas, acne, etc.

The advantages of the therapeutical composition against dermatosis according to the invention are the following: The drug acts upon the anomalies for elimination of the pathological aberrations in the metabolism. Wide-scoped and targeted support of the trophics is achieved in accordance with the specific requirements for correction of the anomalies. The drug has marked anti-inflammatory, soothing, anti-allergic, antimicrobial, targeted trophic, antioxidant and antitoxic effect. It regulates and stimulates the metabolic processes, normalizing the hyperproliferation (cytostatically) and the regeneration of the dermis tissues.

### EXAMPLES

The present invention will now be illustrated by the following non-limiting Examples.

### Example 1.

Water-ethanol-oil extract is obtained from: Calendula officinalis L. 3 parts by weight and Hypericum perforatum L. 2 parts by weight.

The extract contains low-molecular peptides and free amino acids to a total amount of 4.5%, more specifically histidine, cystine, serine, alanine, valine, leucine, isoleucin, phenylalanine, glycine, lysine, proline, tyrosine, glutamine, asparagine, methionine; unsaturated fatty acids 5%, more specifically linoleic acid, linolenic acid and oleic acid; vitamins 4.5%, including retinoides 2%; minerals and microelements 5%.

The method for preparing the extract is the following:
70% fresh plant material and 30% dried plant material from Calendula officinalis L. and Hypericum perforatum L. is used. The particle size of the dried plant material is from 1 to 10 mm, and of the fresh plant material - from 10 to 20 mm. The extraction is made with water, 30% ethanol and 45% Olium olivarum. During the extraction constant contact between the components is ensured by stirring. The extraction is carried out step-by-step with duration as follows: Water extraction: 1/2 of the plant material intended for extraction is extracted with water in a ratio 1:5 for 20 days at a temperature of 18°C. The extract is decanted and 5 parts by weight of water are added to the drug. The extraction continues for another 25 days at a temperature up to 25°C and 5 days at a temperature of 30°C. The extracts are mixed, filtered, concentrated and stabilized.

Extract of 1/4 of the plant material is extracted with 30% ethanol in a ratio 1-6 for 30 hours at a temperature of 25°C. The extract is decanted and the residue is further extracted with 45% ethanol by means of a reflux condenser for 8 hours at a temperature of 78°C. It is evaporated to a dry extract. The alcoholic extracts are dried under vacuum to obtain a dry extract.

Oil extraction: the remaining 1/4 of the plant material is extracted with olive oil in a ratio 1:8 during 5 days at a temperature of 30°C, 5 days at a temperature of 25°C and 5 days at a temperature of 20°C. The extracts are mixed.

### Example 2

To the water-ethanol-oil extract in Example 1 is added an extract of Licopodim Clavalim L. In a ratio of 1.2 parts by weight to the total quantity of Calendula officinalis L. and Hypericum perforatum L. Licopodium Clavatum L. provides anti-itching, painkilling, anti-rheumatic, anti- hyperproliferation action.

### Example 3

To the water-ethanol-oil extract in Example 1 is added an extract of bees' pollen in a ratio of 0.5 parts by weight and an extract of propolis 0.5 parts by weight to the total quantity of Calendula officinalis L. and Hyperlcum perforatum L. These apian products extend the scope of the trophic, anti-inflammatory, regenerative and metabolism-regulatory effect.

### Examples 4 - 25

Table 1 describes extracts of Calendula officinalis L. and Hypericum perforatum L., obtained by the method described in Example 1, included in a pharmaceutical formulation. In Examples 7-21 the unguent contains extracts of the medicinal herbs listed in the table, obtained by known methods.

### Example 26

613 patients suffering from different forms of psoriasis were treated. The cycle of treatment was 55 days on the average.

483 results were recorded in an average cycle of 55 days.

The control tests were made on the 15, 30, 45, 60 and 90 day. In 148 patients were registered 22 accompanying diseases, among which hypertonia - 53 patients, allergy - 10 patients, diabetes - 17 patients, hepatitis - 19 patients, podagra - 4 patients, ulcers - 9 patients. Hereditarily defective with psoriasis were 128 patients.
- 47 patients had complications - atropathy related to psoriasis.
- 268 patients had complete recovery with stable result.
- 126 patients showed clear improvement.
- In 52 patients till the 55 day partial improvement was observed.
- In 37 patients serious counter indications were found.

The majority of the last two groups had the disease for more than 15 years. They suffered the cumulative effect of accompanying diseases, hereditary defectiveness, and application of unsuitable therapy over large periods. This requires extension of the treatment to 90 days on the average and the application of an additional complex therapy. A large part of them show prospects for positive results.

The remaining 130 patients did not complete the average 55-day cycle of treatment. Some of them discontinued their treatment for objective reasons and did not follow the recommendations and prescriptions of the medical team, which is the reason for prolongation of the treatment. Nevertheless, this group also shows a similar picture to that of the group of 483 patients.

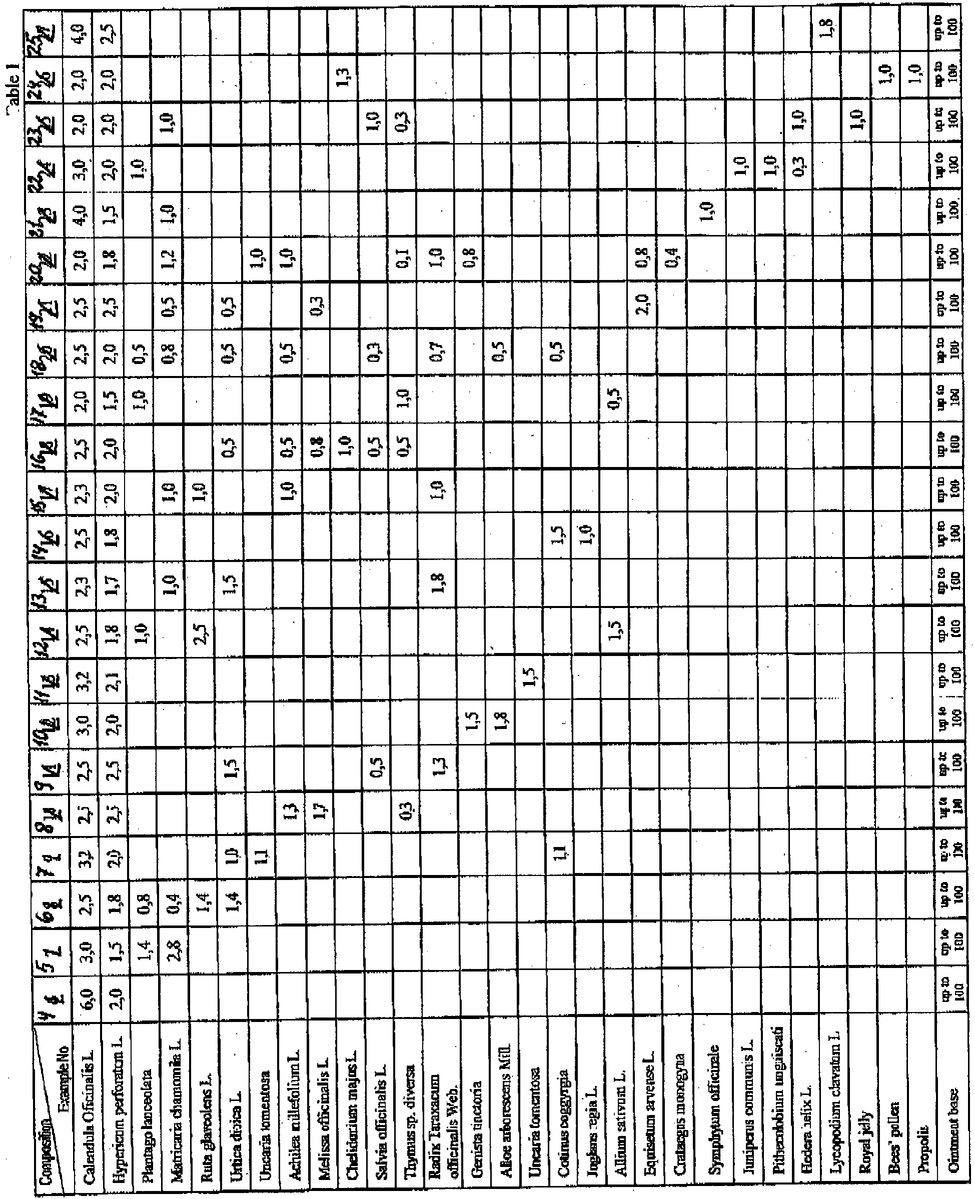

## Claims

1. Therapeutical composition for use in the treatment of dermatosis, in particular psoriasis, containing an extract of the medicinal herbs Calendula officinalis L. and Hypericum perforatum L., **characterized in that** the plant material from Calendula officinalis L. and Hypericum perforatum L. are in a ratio from 1:1 to 3:1 parts by weight and the extract includes low-molecular peptides and free amino acids to a total amount of 0.5-6.0 % by weight of the extract dry mass, more specifically histidine, cystine, serine, alanine, valine, leucine, isoleucine, phenylalanine, glycine, lysine, proiine, tyrosine, glutamine, asparagine and methionine; unsaturated fatty acids 1.0-10.0% by weight of the extract dry mass, more specifically linoleic acid, linolenic acid and oleic acid; vitamins 1-6% by weight, including retinoides 0.6-3.0% by weight of the extract dry mass; and minerals and microelements 1.0-6.0% by weight of the extract dry mass.

2. Therapeutical composition for use according to claim 1, **characterized in that** the extract may contain additionally extracts of at least one of the medicinal herbs selected from the group: Lycopodium Clavatum L., Matricaria chamomilla L., Ruta glaveolens L., Urtica dioica L., Uncaria tomentosa, Achillea millefolium L., Melissa officinalis L., Chelidonium majus L., Salvia officinalis L., Thymus sp. diversa, Taraxacum officinale Web., Genista tinctoria L., Alloe arborescens Mill, Uncaria tomentosa, Cotinus coggyrgia, Juglans regia L., Allium sativum L., Equisetum arvense L., Crataegus monogyna Jacq., Plantago lanceolata L. Symphytum officinale L., Juniperus communis L., Pithecolobium unguiscati, Hedera helix L.

3. Therapeutical composition for use according to claims 1 and 2, **characterized in that** the ratio of the said medical herbs according to claim 2 to the total mass content of Calendula officinalis and Hypericum perforatum is determined so as the quantities of low-molecular peptides and free amino acids, fatty acids, vitamins, minerals and microelements in the end extract would be higher than the lower content limit of these substances.

4. Therapeutical composition for use according to claims 1 and 2, **characterized in that** to the said extract of Calendula officinalis L. and Hypericum perforatum L. is added an extract of at least one apian product: honey, royal jelly, beeswax, bees' pollen, propolis, in a quantity 10-25% by weight of the said extract of Calendula officinalis L. and Hypericum perforatum L.

5. A method for preparing an extract for use according to claim 1, **characterized in that** 30 to 70 % fresh plant material from Calendula officinalis L. and Hypericum perforatum L. and dried plant material for the remaining percentage up to 100 % are used, where the fresh plant material is of particle size 10-20 mm and the dried plant material is of particle size 1-10 mm and an extraction is carried out in a herb to solvent ratio between 1:5 and 1:25 at a temperature between 10°C and 78°C in condition of stirring, said method comprising the steps of: ½ of the plant material is extracted with water in a ratio 1:6 for a period of 20 days at a temperature of 18°C, the extract obtained is decanted and 5 parts by weight of water are added to the residue, the water extraction is carried out in 2 to 4 phases of total duration of about 50 days, and at the phase borders the extract is stabilized with known preservatives and stabilizers such as esters of parahydrobenzoic acid, nipagin, nipazol, and the extracts obtained are mixed, filtered, stabilized and concentrated; ¼ of the plant material intended for extraction is extracted with organic solvent 30% ethanol in a ratio 1:6 for 24 to 48 hours at a temperature of 25°C, the extract obtained is decanted and the residue is further extracted with 4-6 part by weight of 45% ethanol by means of a reflux condenser for 6 to 12 hours at a temperature of 78°C and the two extracts obtained are filtered, mixed, concentrated and stabilized; the remaining ¼ of the plant material intended for extraction is extracted with vegetable oil such as olive oil in a ratio 1:5 for 5 days at a temperature of 30°C , then for 5 days at a temperature of 25°C, and for another 5 day-period at a temperature of 20°C and the oil extract obtained is stabilized with known antioxidants and finally the obtained water extract, organic solvent extract and oil extract are mixed.

## Patentansprüche

1. Therapeutische Zusammensetzung zur Verwendung zur Behandlung von Dermatose, insbesondere Psoriasis, die einen Extrakt der Heilpflanzen Calendula officinalis L. und Hypericum perforatum L. enthält, **dadurch gekennzeichnet, dass** der Pflanzenstoff aus Calendula officinalis und Hypericum perforatum in einem Verhältnis von 1:1 bis 3:1 Gew.-% besteht und der Extrakt niedermolekulare Peptide und freie Aminosäuren in einer Gesamtmenge von 0,5 bis 6 Gew.-% der Extrakttrockenmasse enthält, insbesondere Histidin, Cystin, Serin, Alanin, Valin, Leucin, Isoleucin, Phenylalanin, Glycin, Lysin, Prolin, Tyrosin, Glutamin, Asparagin und Methionin; ungesättigte Fettsäuren von 1,0 bis 10,0 Gew.-% der Extrakttrockenmasse, insbesondere Linolsäure, Linolensäure und Ölsäure; insgesamt Vitamine 1-6 Gew.-%, enthaltend Retinoiden 0,6-3,0 Gew.-% des Trockenextraktes; sowie Mineralstoffe und Spurenelemente 1,0-6,0 Gew.-% der Extrakttrockenmasse.

2. Therapeutische Zusammensetzung zur Verwendung gemäß Anspruch 1, **dadurch gekennzeichnet, dass** der Extrakt zusätzlich Extrakte aus mindestens einer Heilpflanzen enthalten kann, ausgewählt aus der Gruppe: Lycopodium Clavatum L., Matricaria chamomilla L., Ruta glaveolens L., Urtica dioica L., Uncaria tomentosa, Achillea millefolium L., Melissa officinalis L., Chelidonium majus L., Salvia officinalis L., Thymus sp. diversa, Taraxacum officinale Web, Genista tinctoria L., Alloe arborescens Mill, Uncaria tomentosa, Cotinus coggyrgia, Juglans regia L., Allium sativum L., Equisetum arvense L., Crataegus monogyna Jacq., Plantago lanceolata L., Symphytum officianle L., Juniperus communis L., Pithecolobium unguiscati, Hedera helix L.

3. Therapeutische Zusammensetzung zur Verwendung gemäß Anspruch 1 und 2, **dadurch gekennzeichnet, dass** das Verhältnis der Heilpflanzen gemäß Anspruch 2 zur Gesamtmasse von Calendula officinalis L. und Hypericum perforatum L. so festgelegt ist, dass die Mengen von niedermolekularen Peptiden und freien Aminosäuren, Fettsäuren, Vitaminen, Mineralstoffen, Spurenelementen im Endextrakt entsprechend höher als der untere Grenzgehalt dieser Substanzen sind.

4. Therapeutische Zusammensetzung zur Verwendung gemäß Anspruch 1 und 2, **dadurch gekennzeichnet, dass** dem Extrakt von Calendula officinalis L. und Hypericum perforatum L. Extrakt von mindestens einem Bienenprodukt beigefügt ist: Honig, Gelee Royale, Bienenwachs, Bienenpollen, Propolis, in einer Menge von 10 bis 25 Gew.-% des Calendula-officinalis L.- und Hypericum-perforatum L.-Extrakts.

5. Verfahren zur Herstellung eines Extraktes zur Verwendung gemäß Anspruch 1, **dadurch gekennzeichnet, dass** 30% bis 70% frischer Pflanzenstoff aus Calendula officinalis L.und Hypericum perforatum L. und Trockenpflanzenstoff für die restlichen Prozentsätze bis 100% verwendet werden, wobei der frische Pflanzenstoff eine Partikelgröße von 10-20 mm aufweist und der Trockenpflanzenstoff eine Partikelgröße von 1-10 aufweist; und daß eine Extraktion im Verhältnis von Heilpflanzen zum Lösungsmittel zwischen 1:5 und 1:25, bei einer Temperatur zwischen 10°C und 78°C, unter Rühren durchgeführt wird; wobei das Verfahren folgende Schritte umfasst: ½ des Pflanzengutes wird mit Wasser extrahiert im Verhältnis von 1:6 für die Dauer von 20 Tagen bei Temperatur von 18°C; der so erhaltene Extrakt wird dekantiert und 5 Gew.-% Wasser werden zu dem Rest hinzugefügt, die Wasserextraktion wird in 2 bis 4 Phasen mit einer Gesamtdauer von etwa 50 Tagen durchgeführt, indem der Extrakt an der Phasengrenze mit bekannten Konservierungsmitteln und Stabilisatoren wie Ester von p-Hydroxybenzoesäure, Nipagin, Nipasol stabilisiert wird; die so erhaltenen Extrakte werden gemischt, filtriert und konzentriert; ¼ des zur Extraktion vorgesehene Pflanzengutes wird mit einem organischen 30%-Ethano- Lösungsmittel in einem Verhältnis von 1:6 für 24 bis 48 Stunden bei einer Temperatur von 25°C extrahiert; der so erhaltene Extrakt wird dekantiert und der Rest wird weiter mit 4-6 Gew.-% von 45%-gem Ethanol im Rückflusskühler bei einer Temperatur von 78°C für 6 bis 12 Stunden extrahiert; die hergestellten zwei Extrakte werden filtriert, gemischt, konzentriert und stabilisiert; das restliche 1/4 des für Extraktion bestimmten Pflanzengutes wird mit Pflanzenöl wie Olivenöl in einem Verhältnis von 1:5 für 5 Tage bei einer Temperatur von 30°C, anschließend für 5 Tage bei einer Temperatur von 25°C und schließlich für 5 Tage bei einer Temperatur von 20°C extrahiert; der so erhaltene Ölextrakt wird mit bekannten Antioxidantien stabilisiert und schließlich werden der gewonnene Wasserextrakt, der organische Lösungsmittelextrakt und der Ölextrakt miteinander vermengt.

## Revendications

1. Composition thérapeutique, utilisée pour le traitement de dermatoses, notamment le psoriasis, contenant un extrait des herbes médicinales Calendula officinalis et Hypericum perforatum, **caractérisée par le fait que** le matériel végétal de Calendula officinalis et Hypericum perforatum est en proportions de 1:1 à 3:1 parties pondérales et que l'extrait contient des peptides de bas poids moléculaire et des acides aminés libres en quantité globale de 0.5-6.0% du poids de la masse sèche de l'extrait, plus précisément : histidine, cystine, sérine, alanine, valine, leucine, isoleucine, phénylalanine, glycine, lysine, proline, tyrosine, glutamine, asparagine et méthionine ; des acides gras insaturés de 1.0-10.0% du poids de la masse sèche de l'extrait et plus précisément de l'acide linoléique, de l'acide linolénique et de l'acide oléique; des vitamines au total de 1-6% en poids, y compris des rétinoïdes de 0.6-3.0% du poids de la masse sèche de l'extrait et des minéraux et des micro-éléments de poids de 1.0-6.0% de la masse sèche de l'extrait.

2. Composition thérapeutique à utiliser conformément à la revendication 1, **caractérisée par le fait que** l'extrait peut contenir en complément des extraits d'une plante médicinale au minimum ou de plusieurs plantes médicinales faisant partie du groupe suivant: Lycopodium Clavatum L., Matricaria chamomilla L., Ruta glaveolens L., Urtica dioica L., Uncaria tomentosa, Achillea millefolium L., Melissa officinalis L., Chelidonium majus L., Salvia officinalis L., Thymus sp. diversa, Taraxacum officinale Web., Genista tinctoria L., Alloe arborescens Mill, Uncaria tomentosa, Cotinus coggyrgia, Juglans regia L., Allium sativum L., Equisetum arvense L., Crataegus monoguna Jacq., Plantago lanceolata L. Symphytum officinale L., Juniperus communis L., Pithecolobium unguiscati, Hedera helix L.

3. Composition thérapeutique à utiliser conformément aux revendications 1 et 2, **caractérisée par le fait que** la proportion des plantes médicinales aux termes de la revendication 2 par rapport au poids total de Calendula officinalis et Hypericum perforatum est définie de sorte que les quantités des peptides de faible poids moléculaire et des acides aminés libres, acides gras, vitamines, minéraux et microéléments dans l'extrait final soient supérieures à la limite inférieure de la teneur de ces substances.

4. Composition thérapeutique à utiliser conformément aux revendications 1 et 2, **caractérisée par le fait qu'**à l'extrait mentionné de Calendula officinalis L. et Hypericum perforatum L. est ajouté l'extrait d'au moins un produit des abeilles: miel, gelée royale, cire d'abeille, pollen, propolis, en quantité de 10-25% du poids dudit extrait de Calendula officinalis L. et Hypericum perforatum L.

5. Méthode de préparation de l'extrait à utiliser, conformément à la revendication 1, **caractérisée par le fait que** pour la préparation sont utilisés de 30 à 70 % de matériel végétal frais de Calendula officinalis L. et Hypericum perforatum L.; le reste jusqu'à 100 % est du matériel végétal sec, la dimension des particules dans le matériel végétal frais étant de 10-20 mm et celle des particules dans le matériel végétal sec : de 1-10 mm; l'extraction est réalisée dans des proportions de la plante médicinale par rapport au solvant entre 1:5 et 1:25, à une température entre 10°C et 78°C, dans un processus de mélangement, la méthode indiquée comporte les étapes suivantes : ½ du matériel végétal est extrait à l'eau en proportion de 1:6, pendant une période de 20 jours, à une température de 18°C; l'extrait obtenu est décanté et au reste sont ajoutées 5 parties en poids d'eau; l'extraction à l'eau s'effectue en 2 à 4 phases, elle est d'une durée totale d'environ 50 jours; à la limite entre deux phases, l'extrait est stabilisé avec des conservateurs et des stabilisants connus comme des esters de l'acide parahydroxybenzoïque, la nipagine, le nipasol et les extraits obtenus sont mélangés, filtrés, stabilisés et concentrés; ¼ dru matériel végétal, destiné à l'extraction, est extrait avec un solvant organique - 30 % d'éthanol en proportion de 1:6, pendant 24 à 48 heures, à une température de 25°C; l'extrait obtenu est décanté et le reste est de nouveau soumis à l'extraction à reflux avec 4 à 6 parties en poids d'éthanol 45%, pendant 6 à 12 heures, à une température de 78°C et les deux extraits obtenus sont filtrés, mélangés, concentrés et stabilisés; le reste d'¼ du matériel végétal, destiné à l'extraction, est extrait à l'aide d'une graisse végétale comme l'huile d'olive, en proportion de 1:5, pendant 5 jours, à une température de 30°C et puis, pendant les 5 jours suivants, à une température de 25°C et puis, pendant les 5 jours suivants, a une température de 20°; l'extrait huileux obtenu est stabilisé à l'aide d'antioxydants connus et enfin, on procède au mélangement des extraits obtenus: l'extrait aqueux, l'extrait de solvant organique et l'extrait huileux.
